(19) Europäisches Patentamt
European Patent Office
Office européen des brevets

(11) Veröffentlichungsnummer: **0 035 472**
**A1**

## (12) EUROPÄISCHE PATENTANMELDUNG

(21) Anmeldenummer: **81810059.6**

(22) Anmeldetag: **25.02.81**

(51) Int. Cl.³: **C 07 C 149/20**, C 07 C 149/18, C 08 K 5/37

(30) Priorität: **03.03.80 CH 1664/80**

(43) Veröffentlichungstag der Anmeldung: **09.09.81 Patentblatt 81/36**

(84) Benannte Vertragsstaaten: **DE FR GB IT**

(71) Anmelder: **CIBA-GEIGY AG, Patentabteilung Postfach, CH-4002 Basel (CH)**

(72) Erfinder: **Rosenberger, Siegfried, Im Gehracker 11, CH-4125 Riehen (CH)**
Erfinder: **Evans, Samuel, Dr., Schützenrain 3, CH-4125 Riehen (CH)**
Erfinder: **Gilg, Bernard, Dr., Route de Bâle, F-68300 Saint-Louis (FR)**

# (54) Mercaptophenole und ihre Verwendung als Stabilisatoren.

(57) Die erfindungsgemäßen Verbindungen entsprechen der Formel I

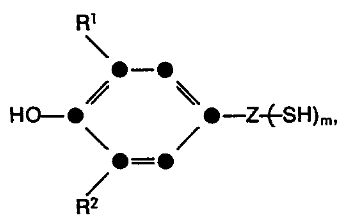

(I)

in der m eine der Zahlen 1 oder 2 darstellt, $R^1$ und $R^2$ gleich oder verschieden sind und vorzugsweise einen tert.-Butylrest bedeuten. Z steht für einen 2- oder 3wertigen organischen Rest, so beispielsweise für

$$-CH_2SCH_2CO \bullet O \bullet CH_2CH_2-.$$

Die Verbindungen sind besonders zum Stabilisieren von Polymeren unter Aufpfropfung geeignet.

EP 0 035 472 A1

0035472

CIBA-GEIGY AG
Basel (Schweiz)

3-12734/+

Mercaptophenole und ihre Verwendung als Stabilisatoren

Die Erfindung betrifft neue Mercaptophenole, welche als Stabilisatoren für organisches Material Verwendung finden.

Es sind bereits Phenolgruppen und Mercaptogruppen enthaltende Stabilisatoren für organisches Material, insbesondere für Polymere, bekannt, welche beispielsweise nach Einmischen in das zu stabilisierende Material ihre Wirkung entfalten. Im Falle der Stabilisierung von Polymeren, insbesondere gegen Oxidation, kann die Stabilisierung sehr vorteilhaft in der Weise erfolgen, dass man den jeweiligen Stabilisator in Gegenwart eines Radikalbildners auf das Polymer aufpropft. Derartige Stabilisator-Fixierungen auf Polymeren sind ausführlich in der DE-OS 2 509 654 beschrieben. Als weitere einschlägige Publikationen sind eine Notiz in Chemical Age 17/24, August 1979, Seite 5, und eine Abhandlung in British Plastic and Rubber, April 1977, Seiten 25-26, anzuführen. In der letztgenannten Publikation werden auf Seite 26 noch weitere Publikationen von Gerald Scott in verschiedenen Zeitschriften angegeben.

Ausserdem ist auch noch auf eine japanische Publikation "Network bound antioxidants in diene rubber" in Nippon Gomu Kyokaishi 1977, 50(8), 548-552, (Chem. A. 87, (1977) 44 (168997a) und die DE 2 659 406 hinzuweisen.

Die Bindung von Stabilisatoren an die Polymeren, welche über Schwefelbrücken erfolgt, bringt folgende Vorteile mit sich:

a) Die Stabilisatoren gehen weder durch Verflüchtigung noch durch Extraktionsvorgänge verloren.

b) Niedere oder fehlende Migration bringt Vorteile toxikologischer Art mit sich.

Die erfindungsgemässen Mercaptophenole zeigen nach ihrer Aufpfropfung auf vorgegebene Polymere bessere Wirksamkeiten, verglichen mit den vorbekannten, ähnlichen Verbindungen.

Gegenstand der Erfindung sind Verbindungen der Formel I

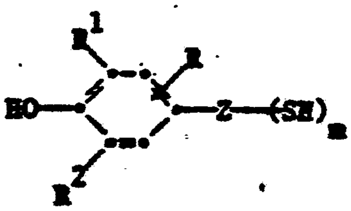 (I) ,

in der m eine der Zahlen 1 oder 2 darstellt, $R^1$ und $R^2$ gleich oder verschieden sind und einen geradkettigen oder verzweigten Alkylrest mit 1 bis 18 C-Atomen, einen Cycloalkyl-, Aryl-, Alkaryl- oder Aralkylrest bedeuten, wobei $R^2$ im Gegensatz zu $R^1$ auch für H stehen kann, in der R einen Alkylrest mit 1 bis 12 C-Atomen oder H bedeutet, in der Z $-OR^6-$, $-NH-R^6-$, $-A(-O-R^6-)_m$, $-A(-NH-R^6-)_m$, $-B-CO\cdot C_nH_{2n}-$,

$-C_qH_{2q}-$, 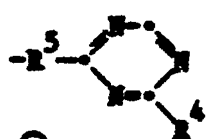 , $-C_2H_{2c}COO^{\ominus}\overset{\oplus}{N}H_3C_nH_{2n}-$, $-\overset{\oplus}{N}H_3\ \overset{\ominus}{O}CO\cdot C_nH_{2n}-$ ,

$C_2H_{2c}NH_3^{\oplus}\ \overset{\ominus}{O}CO\cdot C_nH_{2n}-$ ,

und nur bei m=2, 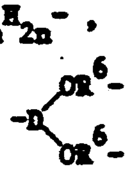 oder 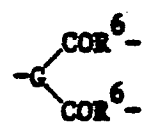 darstellt,

wobei A bei m = 1 einen der 2-wertigen Reste aus der Reihe

$-C_tH_{2t}SC_nH_{2n}\cdot CO-$, $-C_nH_{2n}\cdot COO\cdot C_tH_{2t}SC_nH_{2n}CO-$, $-C_tH_{2t}CO\cdot O\cdot C_nH_{2n}S-C_sH_{2s}\cdot CO-$, $-OC_nH_{2n}CO-$,

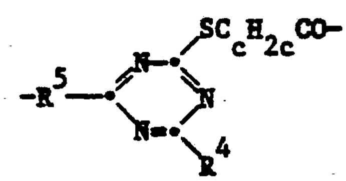 ,

BAD ORIGINAL

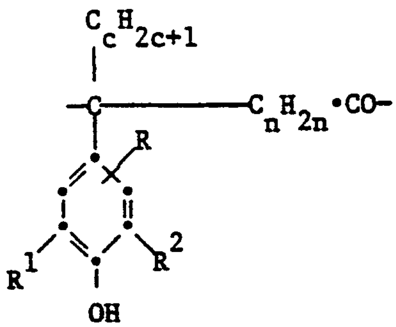

und

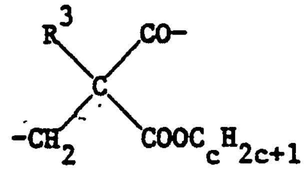

oder aber bei m = 2

einen 3-wertigen Rest

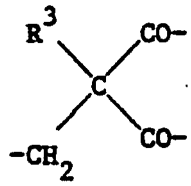

,

B einen Rest aus der Gruppe -O-, -NH-, $-C_nH_{2n}O-$, $-C_nH_{2n}NH-$,

$-C_tH_{2t}SC_nH_{2n}O-$, $-OC_cH_{2c}-$ , $-CH_2SC_nH_{2n}OC_tH_{2t}-$, $-CH_2SC_nH_{2n}-$,

$-C_cH_{2c}CONHC_nH_{2n}-$, $-C_cH_{2c}CONHC_nH_{2n}-NH-$, $-C_cH_{2c}CONH-$ 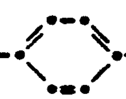 -NH-,

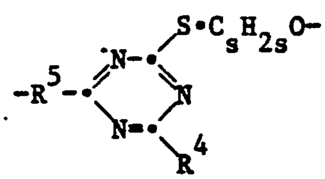

, $-C_nH_{2n}CO{\cdot}O{\cdot}C_tH_{2t}NH-$ , $-R^5-C_nH_{2n}O-$ ,

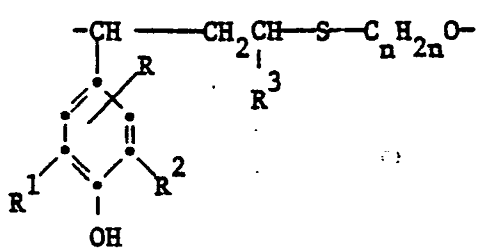

,

D den Rest

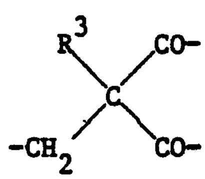

und

G den Rest 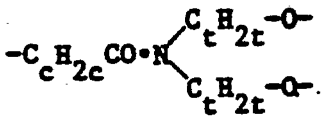

bedeuten, und wobei
c, n, r, s und t gleich oder verschieden sind und für eine Zahl von 1 bis 12 stehen, q für eine Zahl von 2 bis 12 steht, mit der Massgabe, dass die einzelnen Indizes c, n, q, r, s und t ebenfalls gleich oder verschieden sein können, wenn dieselben mehrmals in gleichen oder verschiedenen Molekülgruppen der Verbindung vorkommen, $R^3$ einen Alkylrest mit 1 bis 18 C-Atomen, den Rest

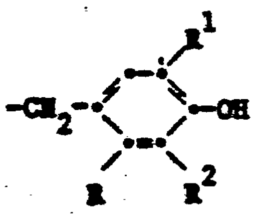 oder H und $R^4$ einen der Reste

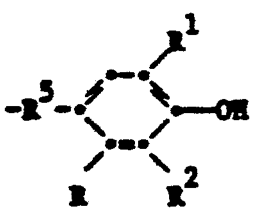 ,

$-R^5-C_pH_{2p+1}$ oder -SH bedeuten, wobei p eine Zahl von 1 bis 18 darstellt, $R^5$ für einen der Reste -NH-, -O- oder -S- und $R^6$ für einen durch Aryl- oder -OH substituierten oder unsubstituierten Alkylenrest mit 2 bis 18 C-Atomen, welcher auch durch -O- oder -S-Brücken unterbrochen sein kann, steht.

$R^1$ und $R^2$ können folgende Alkylreste bedeuten: z.B. Methyl, iso-Propyl, n-Butyl, sec.-Butyl, tert.-Butyl, tert.-Amyl, 2-Aethylhexyl, n-Octyl, tert.-Octyl, n-Dodecyl, 1,1,7,7-Tetramethyl-octyl, n-Octadecyl. α-Verzweigte Alkyl-Radikale mit 3 bis 8 C-Atomen sind bevorzugt.

$R^1$ und $R^2$ können Cycloalkyl bedeuten, wie z.B. Cyclopropyl, Cyclopentyl, Cyclohexyl, Cycloheptyl, Cyclooctyl oder Cyclododecyl. Bevorzugt sind Cyclopentyl und Cyclohexyl.

Stehen $R^1$ und $R^2$ für Aralkyl, so kommen Benzyl, α-Phenyläthyl und 2-Phenyl-iso-propyl in Frage. Bevorzugt sind α-Phenyläthyl und 2-Phenyl-iso-propyl.

Stehen $R^1$ und $R^2$ für Alkaryl, so kann es beispielsweise Tolyl, Xylyl, 2,6-Diäthylphenyl oder 4-tert.-Butylphenyl sein.

Als Aryl stehen für $R^1$ und $R^2$ beispielsweise Phenyl, α-Naphthyl oder β-Naphthyl. Bevorzugt ist Phenyl.

$R^3$ kann dieselben Reste bedeuten wie $R^1$ und $R^2$, wenn sie Alkyl mit 1 bis 18 C-Atomen darstellen.

Bevorzugt bedeuten in Formel I m die Zahl 1 und t, n, r und s unabhängig voneinander eine der Zahlen 1, 2 oder 3 und q 3. Vorzugsweise bedeuten mindestens $R^1$ oder $R^2$ einen tert.Butylrest. Noch mehr bevorzugt bedeuten beide Reste ($R^1$ und $R^2$) einen tert.Butylrest.

Eine besondere Vorzugsform der Erfindung stellen solche Verbindungen der Formel I dar, in der m eine der Zahlen 1 oder 2 darstellt, $R^1$ und $R^2$ gleich oder verschieden sind und einen tert.Butylrest, einen Methylrest, einen Isopropylrest, einen Phenylrest oder den Rest

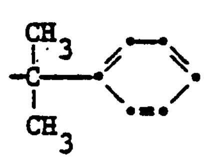

BAD ORIGINAL

bedeuten, in der Z entweder

$-A(-OCH_2CH_2-)_m$ oder $-B-CO \cdot CH_2-$ oder $-C_qH_{2q}-$ oder $-R^5-$ 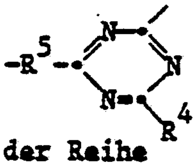 darstellt, wobei A bei m = 1 einen der 2-wertigen Reste aus der Reihe $-CH_2SCH_2CO-$, $-(CH_2)_3SCH_2CO-$, $-CH_2CO \cdot O(CH_2)_3SCH_2CH_2CO-$ und $-OCH_2CO-$ oder aber bei m = 2 einen 3-wertigen Rest

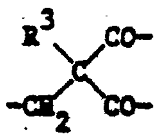

und B einen Rest aus der Gruppe

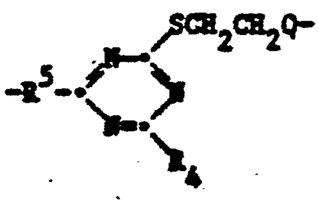 oder $-R^5-CH_2CH_2O-$

bedeuten und wobei q für eine der Zahlen 2 oder 3, $R^3$ für einen Alkylrest mit 1 bis 18 C-Atomen, $R^4$ für einen der Reste

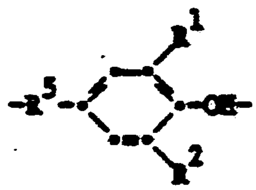 oder $-R^5-C_pH_{2p}$,

wobei p eine Zahl von 1 bis 6 bedeutet, und $R^5$ für einen der Reste -NH-, -O- oder -S- steht.

Eine weitere Vorzugsform der Erfindung stellen Verbindungen der Formel I, in der Z einen der Reste

$-C_qH_{2q}-$ ,

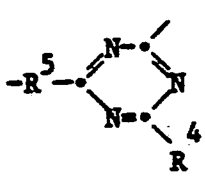 ,

BAD ORIGINAL

$-OR^6-$ ,

$$-C_cH_{2c}COO^{\ominus}\ \ {}^{\oplus}NH_3\ C_nH_{2n}-$$

oder

$$-{}^{\oplus}NH_3\ \ {}^{\ominus}OCOC_nH_{2n}-$$

darstellt, dar. Beispiele für derartige Verbindungen und besonders bevorzugt sind die folgenden:

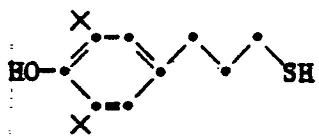 , 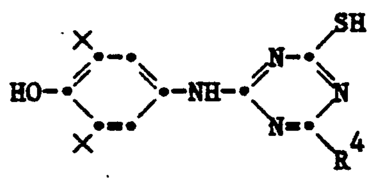 ,

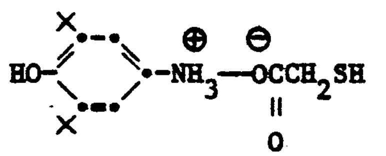 ,

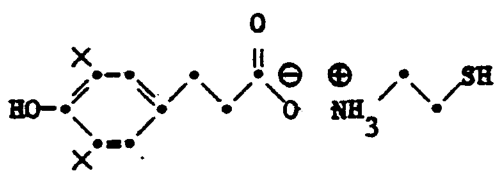 .

Eine weitere Vorzugsform der Erfindung stellen Verbindungen der Formel I dar, in der Z den Rest $-A-OR^6-$ bedeutet, wobei A für einen der Reste

$$-C_tH_{2t}COOC_nH_{2n}S-C_sH_{2s}\cdot CO-$$ ,

$$-C_tH_{2t}SC_nH_{2n}CO-$$ ,

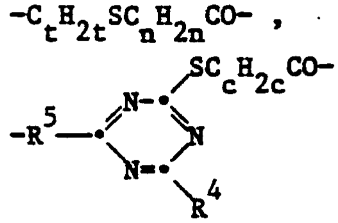

$-OC_nH_{2n}CO-$ oder

BAD ORIGINAL

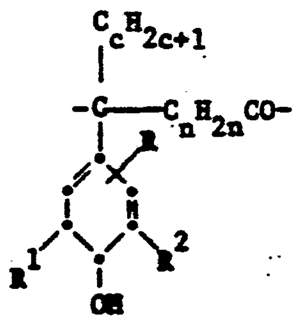

steht.

Besonders bevorzugte Beispiele für diese Verbindungen sind die folgenden:

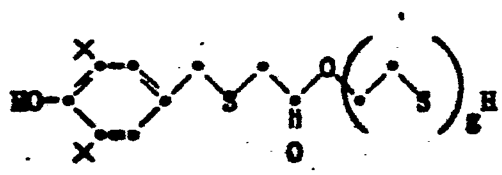

(g = 1 - 4) ,

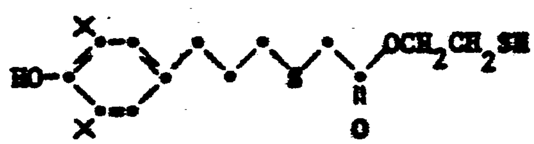

,

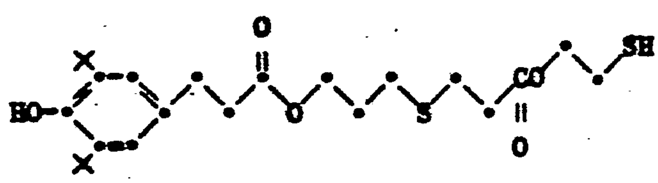

,

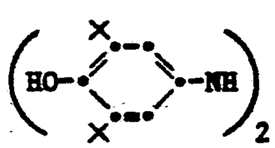 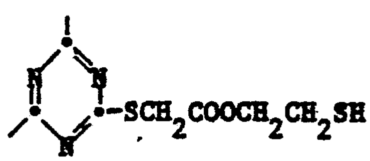

,

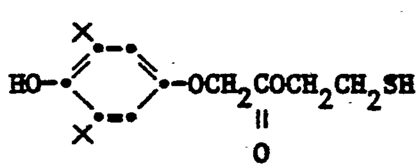

,

BAD ORIGINAL

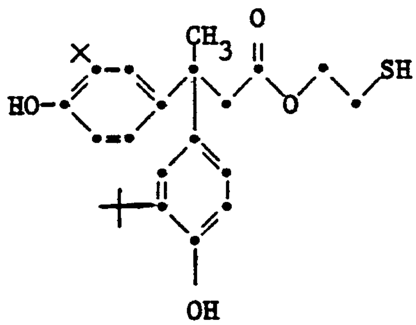

Eine weitere Form der Erfindung stellen Verbindungen der Formel I dar, in der Z den Rest 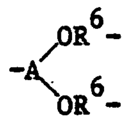 bedeutet, wobei A für den 3-wertigen Rest 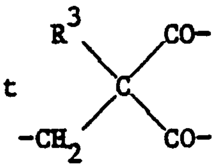 steht.

Besonders bevorzugte Beispiele für diese Verbindungen sind die folgenden:

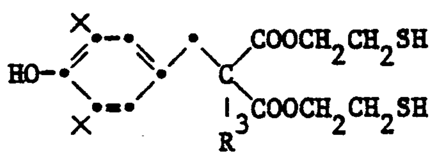 , 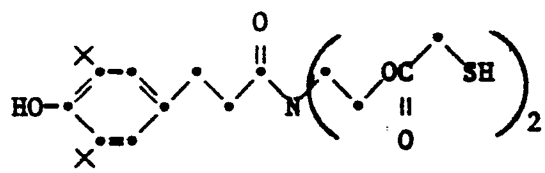 .

Eine weitere Vorzugsform der Erfindung stellen Verbindungen der Formel I dar, in der Z den Rest $-B-CO\cdot C_nH_{2n}-$ bedeutet, wobei

B für einen der Reste -O-, -NH-,

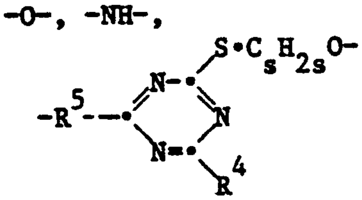 ,

$-C_tH_{2t}SC_nH_{2n}O-$ ,

$-C_cH_{2c}\cdot CO\cdot NHC_nH_{2n}-NH-$ ,

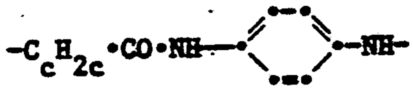 ,

$-C_nH_{2n}NH-$ ,

$-C_nH_{2n}CO\cdot O\cdot C_tH_{2t}NH-$ und

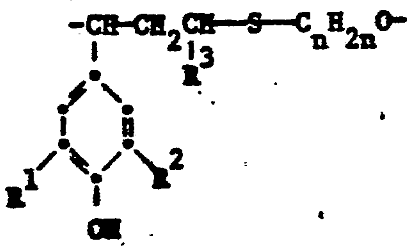

steht.

Besonders bevorzugte Beispiele für diese Verbindungen sind die folgenden:

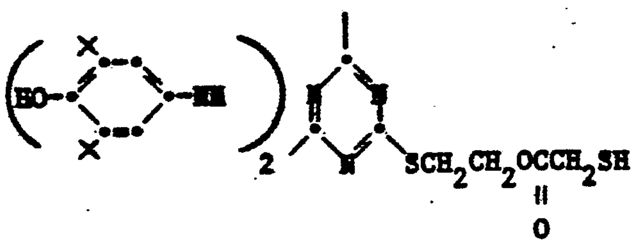 ,

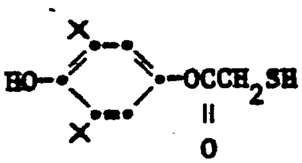 , 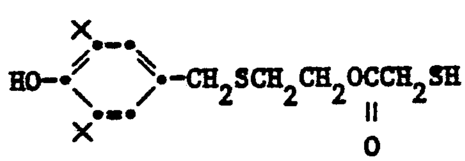 ,

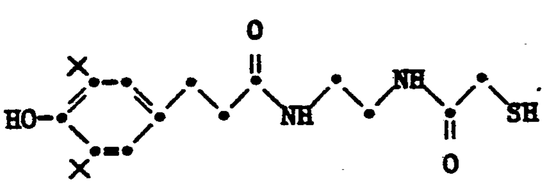 , 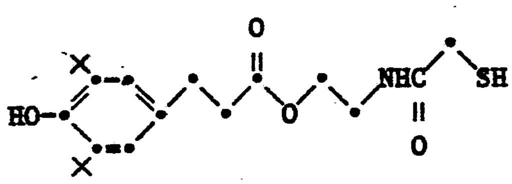 ,

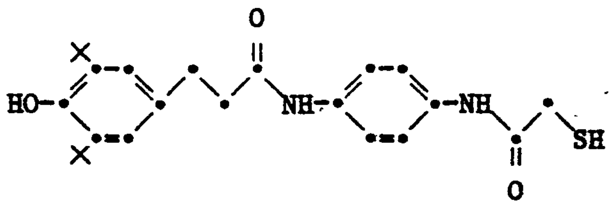
.

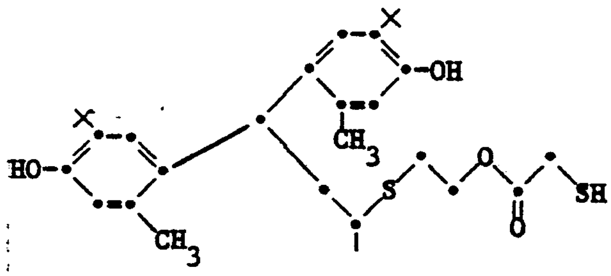
,

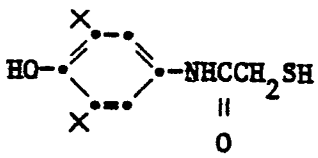
,

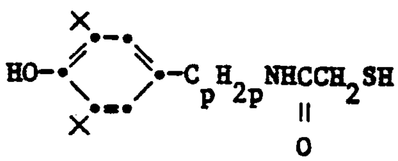
.

Ganz besonders bevorzugte Verbindungen der Formel I sind solche, welche folgende Strukturen aufweisen:

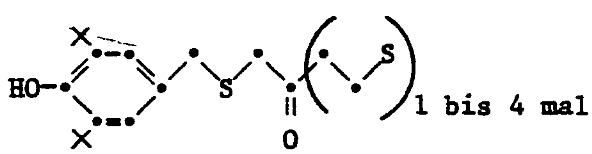
und

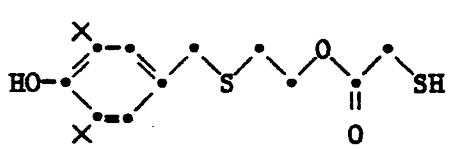
und

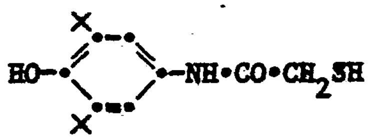

Die Herstellung der erfindungsgemässen Verbindungen erfolgt nach bekannten Verfahren. In der nachfolgenden Zusammenstellung sind bevorzugte Verbindungen der Formel I aufgeführt, wobei gleichzeitig jeweils die Herstellungsmethode formelmässig erläutert wird.

(Die angegebenen Verfahren gelten auch für analoge Substanzen im erweiterten Sinne der Grundformeln bzw. Oberbegriffe).

a) 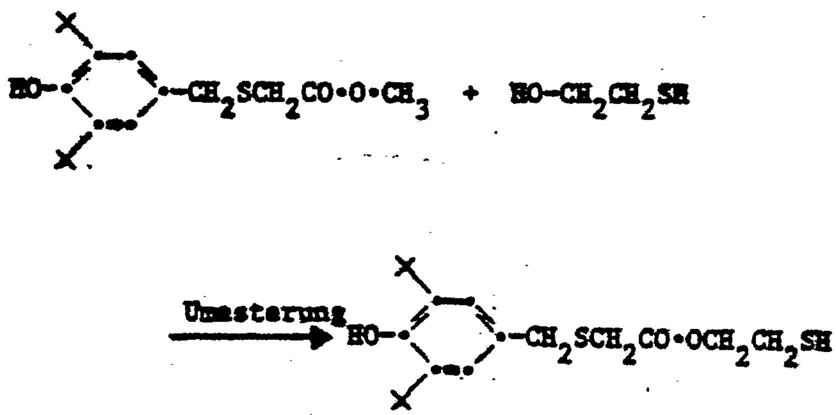


b) 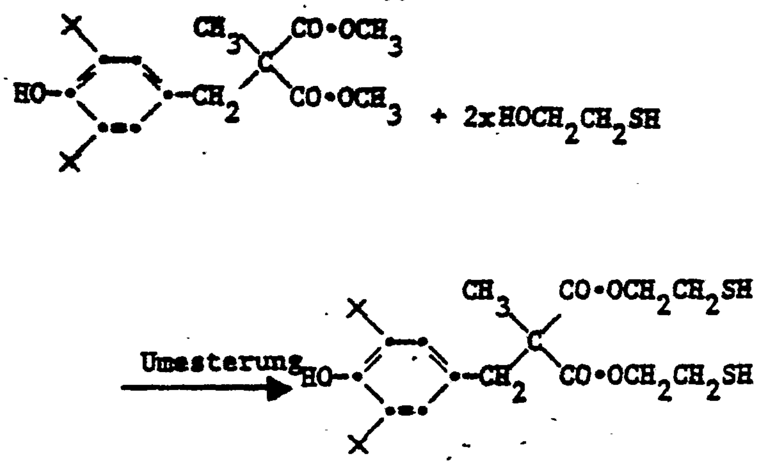


c) 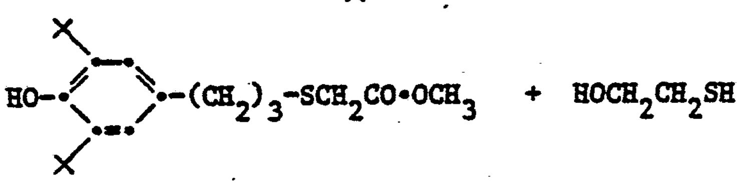

BAD ORIGINAL

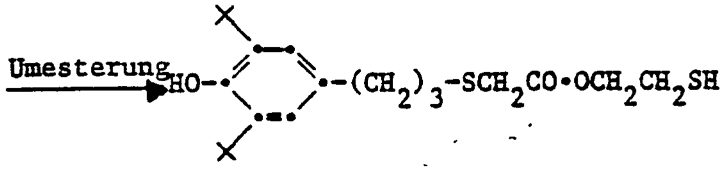


d)
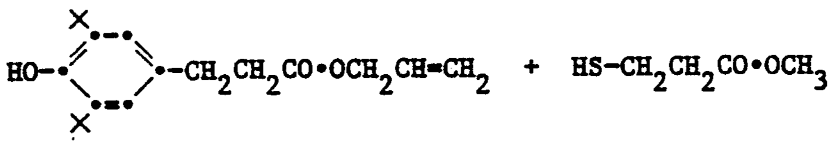

(Radikalische Addition)

(1.Stufe)
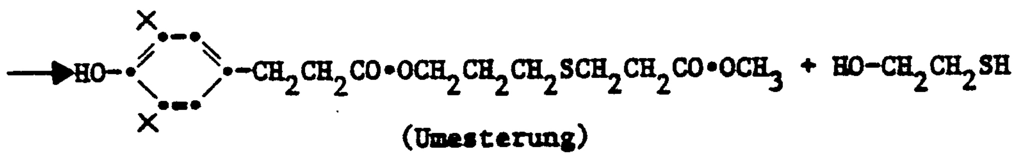

(Umesterung)

(2.Stufe)
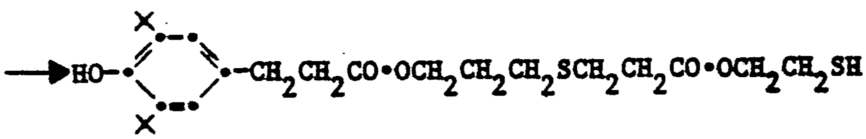

$d_1$)
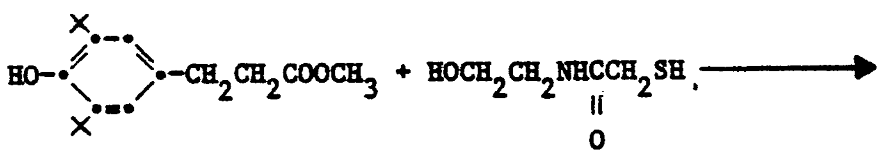

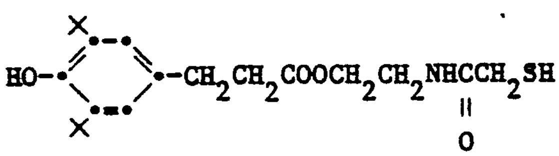

$d_2$)

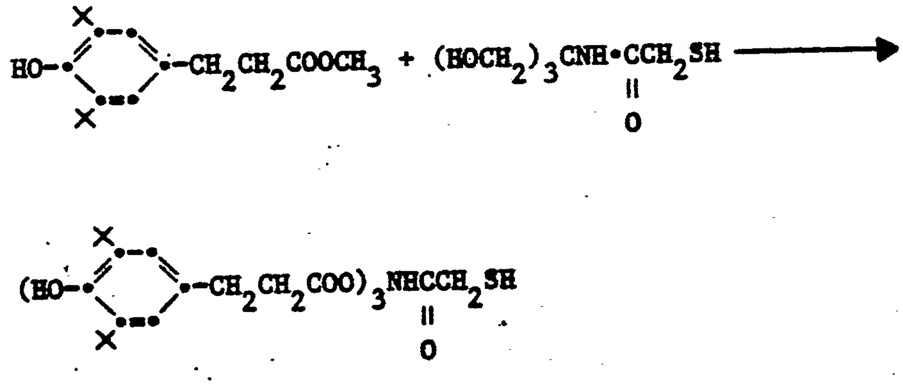

$d_3$)

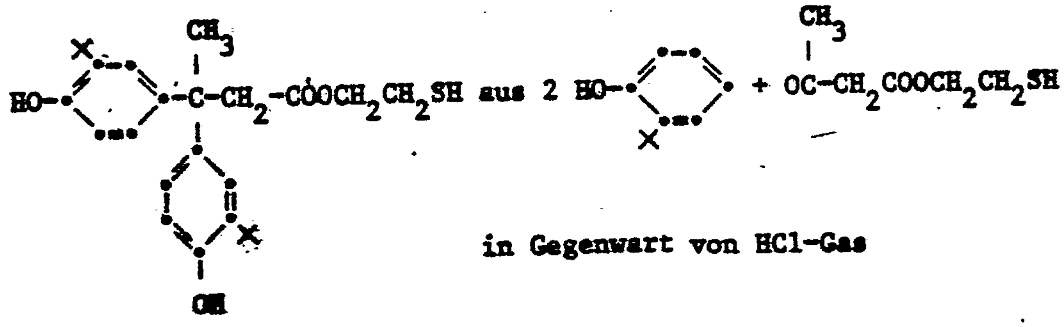


e)

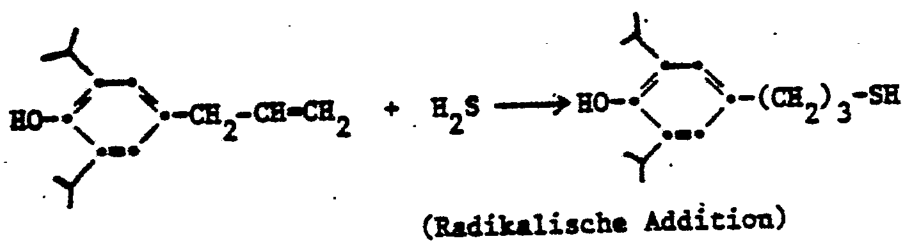

(Radikalische Addition)

f)

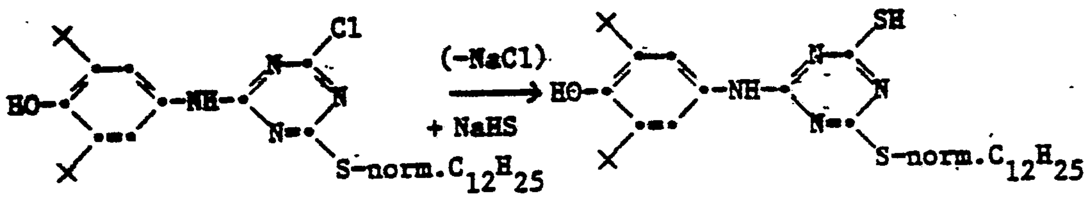


BAD ORIGINAL

g) 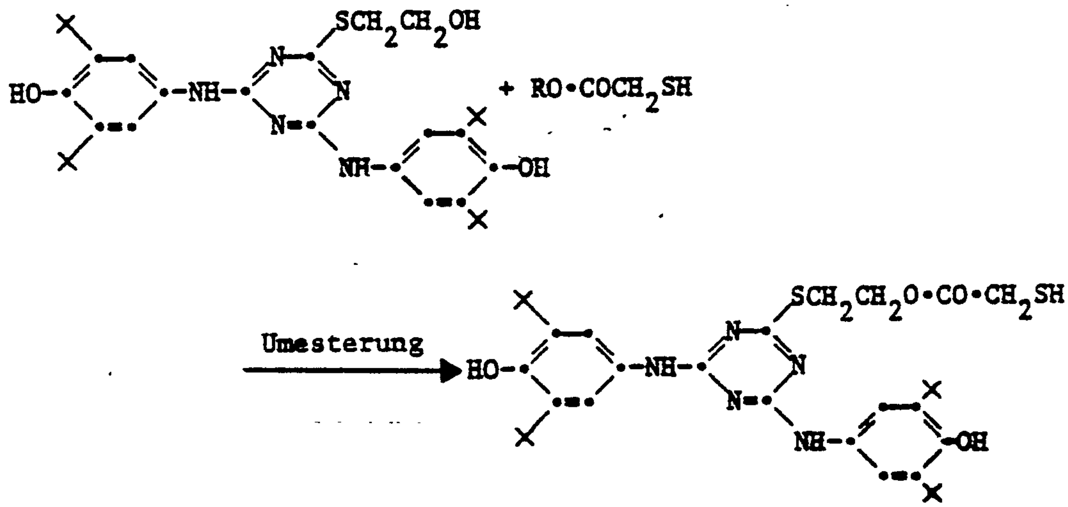


h) 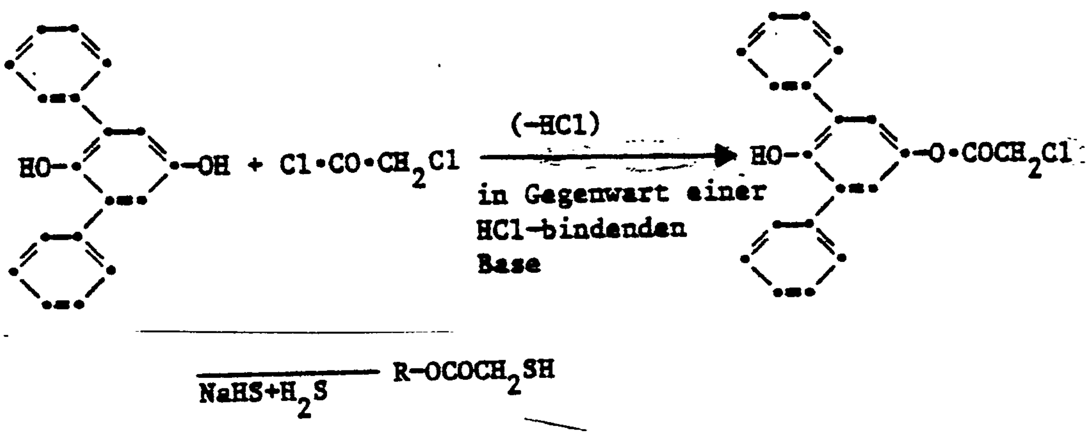


i) 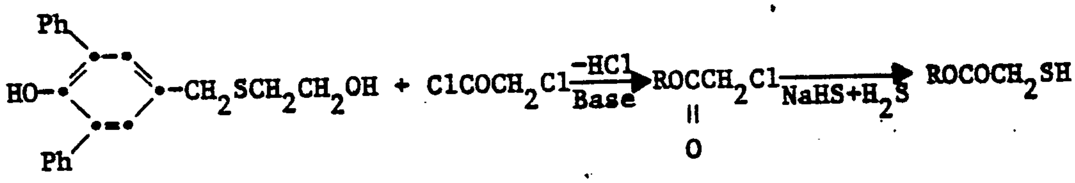


j) 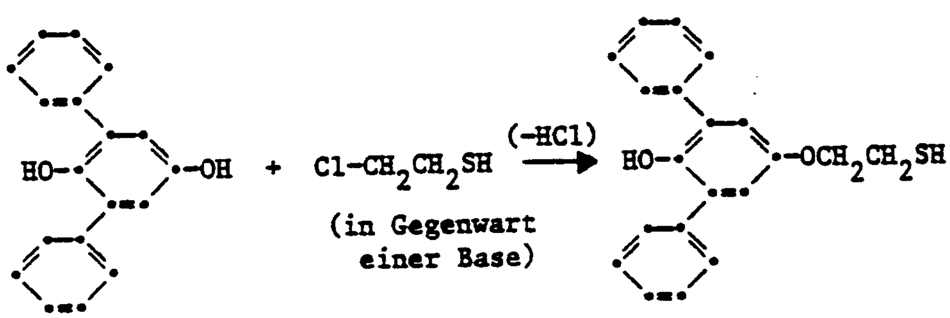


BAD ORIGINAL

j)1) 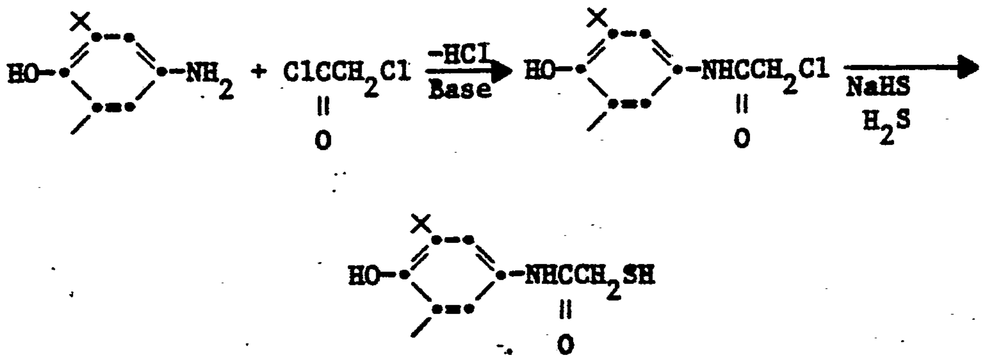


k) 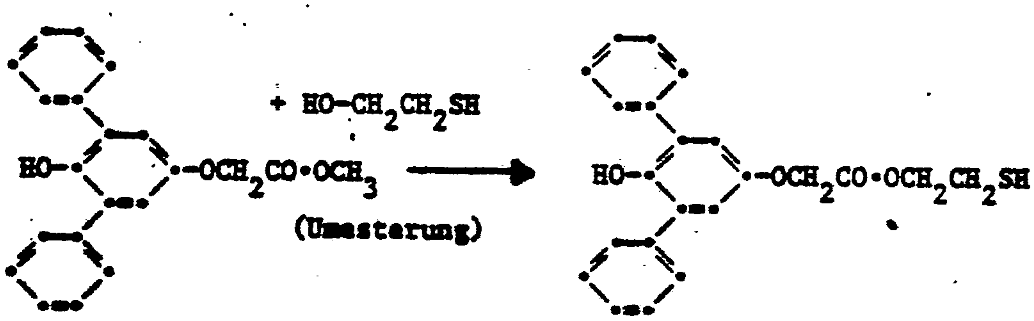


1) 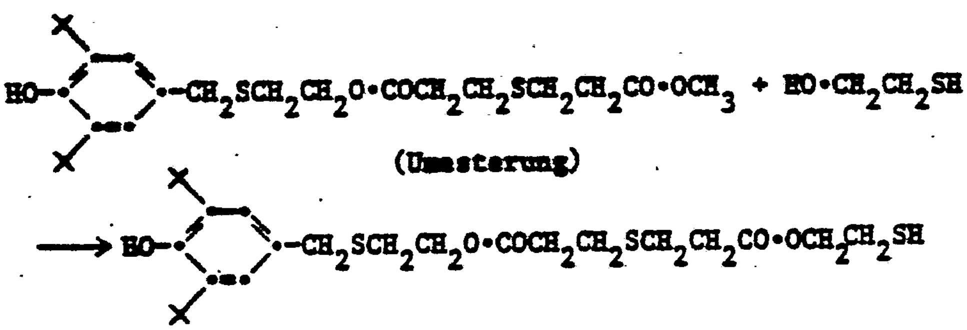


Als Ausgangsstoffe für die Herstellung der erfindungsgemässen Verbindungen werden bekannte Substanzen verwendet, deren Herstellung dem Fachmann ebenfalls bekannt ist.

Weiterer Gegenstand der Erfindung ist die Verwendung von Verbindungen der Formel I oder bekannter Verbindungen der Formeln II oder III

BAD ORIGINAL

0035472

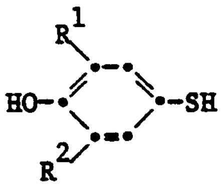 (II) 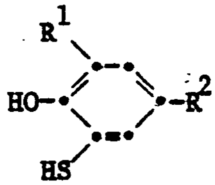 (III) ,

als Stabilisatoren für organisches Material, insbesondere zur Herstellung von stabilisierten Polymeren durch Aufpfropfen der Verbindungen auf die Polymeren.

Bei den als Stabilisatoren verwendeten Verbindungen handelt es sich im wesentlichen um die erfindungsgemässen Verbindungen der Formel I und insbesondere um die vorher angeführten Vorzugsformen der erfindungsgemässen Stoffe. Unter die verwendeten Stabilisatoren fallen aber auch die beiden bekannten Verbindungen der Formeln

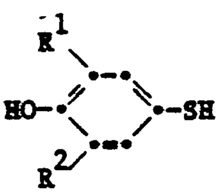 und 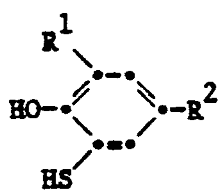 .

Die Verfahren zur Herstellung dieser bekannten Verbindungen sind bekannt, und es erübrigt sich somit hier, Näheres darüber mitzuteilen.

Das Aufpfropfen der erfindungsgemässen Mercaptophenole auf die vorgegebenen Polymeren erfolgt in der Weise, dass die Polymeren mit den Mercaptophenolen in Gegenwart eines Radikalbildners umgesetzt werden. Die Umsetzung kann in wässeriger Emulsion oder Suspension, in Lösung oder in der Masse erfolgen. Vorteilhaft werden für die Reaktion solche Bedingungen angewandt, die denjenigen der Herstellung des Polymeren ähnliche sind. Eine besondere Bedeutung kommt hier der Pfropfung in wässerige Emulsion zu.

Als Radikalbildner kommen organische Peroxide, Hydroperoxide oder Persulfate und deren Kombinationen mit Aminen oder $Fe^{2+}$-Salzen, vorzugsweise Azoverbindungen in Frage. Das Gewichtsverhältnis von Stabilisator zu Radikalbildner ist nicht kritisch und beträgt 100:1 bis 0,25:1.

Die Umsetzung der Stabilisatoren kann mit all den Polymeren erfolgen, welche in der DE-OS 2 509 654 aufgezählt worden sind. So folgende Polymere: Polyolefine, Polystyrol, Polyvinylchlorid, Polyamide, Polyester, natürliche oder synthetische Kautschukarten, Aethylen-Propylen-Kautschuk, Styrol-Butadien, Acrylnitril-Butadien-Styrol, Polybutydien, Polyisopren, Methacrylat-Butadien-Styrol- und Methylmethacrylat-Butadien-Styrol-Copolymere, besonders in Form von Latices. Natürlich können auch Gemische von Polymeren angewandt werden. Die Polymere besitzen normalerweise ein hohes Molekulargewicht, z.B. so, dass das Polymer film- oder faserbildend ist; aber niedermolekulare Polymere, z.B. Polymere, die noch flüssig sind, können ebenfalls angewandt werden, wenn die Addukte als Zusätze für andere Polymere verwendet werden sollen. Als Latices kommen auch folgende Polymere in Frage: NR, BR, NBR, SBR, CR, MBS, ABS.

Bei der erfindungsgemässen Verwendung unter Aufpfropfung wird das Antioxidans in einer Menge von 0,001 bis 5 %, vorzugsweise 0,1 bis 1 %, bezogen auf das Gewicht des Polymers, zugegeben, wenn die Eigenschaften des Polymers modifiziert werden sollen. Wenn andererseits das Antioxidans zugegeben wird, um ein Addukt herzustellen, das angewandt werden kann, um die Eigenschaften eines anderen Polymers zu modifizieren, können grössere Mengen angewandt werden. Im vorliegenden Falle, wo das Antioxidans ein Thiol ist, ist es möglich, so grosse Mengen wie 300 bis 500 Gew.-%, vorzugsweise 50 Gew.-%, zu verwenden. Die Reaktionstemperaturen liegen zwischen 0 und 200°C, vorzugsweise 50 bis 130°C, dagegen bei Latexsystemen zwischen 40 und 60°C. In einem zweiten Schritt werden diese hochprozentigen Pfropfpolymerisate

mit dem Ausgangspolymeren oder einem andern Polymeren verdünnt. Die Verdünnung kann durch Mischen von Latices oder Lösungen, oder auch durch Eincompondieren des hochprozentigen Pfropfpolymerisates erfolgen.

Mit Hilfe der erfindungsgemässen Verbindungen können durch den beschriebenen Pfropfprozess die folgenden Polymeren stabilisiert werden:

Polypropylen, Polyäthylen, Polyisopren, auch als Naturkautschuk, Polybutadien, Terpolymere von Aethylen, Propylen und einem Dien Aethylen-Propylen-Kautschuk, Styrol-Butadien, Styrol-Butadien-Styrol-Blockcopolymerisate, Styrol-Butadien-Pfropfcopolymere, Acrylnitril-Butadien-Styrol-Pfropfcopolymere, Polychloropren, Acrylnitril-Butadien-Copolymere, Methacrylat-Butadien-Styrol-Pfropfpolymere.

(Die bevorzugten Polymere sind unterstrichen).

Die für den Pfropfprozess verwendeten Ausgangspolymeren können weitere Additive enthalten, wie z.B.

Weitere alkylierte Phenole als Antoxidantien, Lichtschutzmittel, wie 2-(2'-Hydroxyphenyl)-benztriazole, 2-Hydroxybenzophenone, Nickelverbindungen, sterisch gehinderte Ancine, Phosphite, Thioäther, Füllstoffe, Weichmacher, Gleitmittel, Flammschutzmittel, Antistatika.

Weitere Gegenstände der Erfindung sind somit auch noch:

a) Die erfindungsgemässe Verwendung dadurch gekennzeichnet, dass man die Verbindungen der Formel I, II und III in Gegenwart eines Radikalbildner auf ein Polymeres aufpfropft.

b) Durch Verbindungen der Formeln I, II und III stabilisierte organische Polymere.

c) Die so stabilisierten Polymeren, dadurch gekennzeichnet, dass sie als Stabilisatoren Verbindungen der Formel I enthalten.

d) Stabilisierte Polymere, dadurch gekennzeichnet, dass sie die Stabilisatoren aufgepfropft enthalten.

Herstellungsbeispiele

Beispiel 1: S-(3,5-Di-t-butyl-4-hydroxy-benzyl)-thioglykolsäure-5-mercapto-3-thia-pentyl-ester

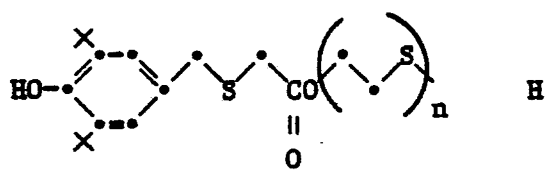

n = 2

32,4 g S-(3,5-Di-t-butyl-4-hydroxy-benzyl)-thioglykolsäure-methylester[1)] werden mit 11,7 g 2-Mercaptoäthanol unter Zusatz von 0,5 ml Tetrabutyl-ortho-titanat 20 Stunden in einer $N_2$-Atmosphäre unter Rühren bei 150°C gehalten. Das Reaktionsgemisch enthält demnach lt. Methoxy-Bestimmung und NMR-Spektrum, nur noch wenig Methylester-Edukt neben einem Gemisch von Mercaptophenolen der obigen Formel mit n = 1-3, aus dem die Verbindung mit n = 2 durch Chromatographie abgetrennt ein gelbliches zähes Harz darstellt, und elementar analytisch sowie durch SH-Titration charakterisiert werden kann.

1) Dieses Edukt ist vorbekannt und kann z.B. hergestellt werden aus äquimolaren Mengen N-(3,5-Di-t-butyl-4-hydroxybenzyl)-N,N-dimethylamin und Thioglykolsäuremethylester durch 20-stündiges Erhitzen auf 130°C,Kristalle aus Hexan, Smp.: 54°C.

<u>Beispiel 2:</u> Ersetzt man im vorangehenden Beispiel den S-(3,5-<u>Di-t-butyl</u>-4-hydroxybenzyl)-thioglykolsäure-methylester durch das homologe S-(<u>3-Methyl</u>-5-t-butyl)-Derivat, so erhält man bei analoger Arbeitsweise das entsprechende Mercaptophenol-Derivat der Formel

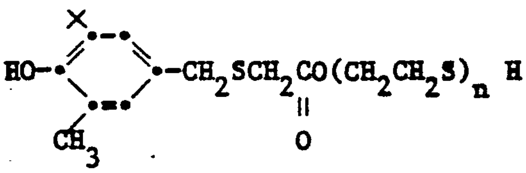

n = 2

als viskoses Oel.

<u>Beispiel 3:</u>

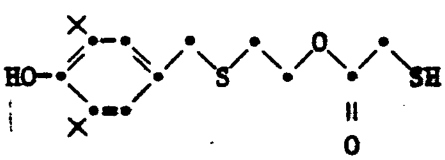

Thioglykolsäure-β-(3,5-di-t-butyl-4-hydroxybenzylmercapto)-äthylester.

22,2 g 4-(β-Hydroxy-äthylmercaptomethyl)-2,6-di-t-butyl-phenol[1)] und 10,6 g Thioglykolsäure-methylester werden unter Zusatz von 0,5 ml Tetrabutyl-ortho-titanat unter Stickstoff und Rühren 6 Stunden auf 135°C erhitzt. Danach sind die Edukte lt. Dünnschicht-Chromatogramm praktisch umgesetzt.

Die Rohschmelze wird säulenchromatographisch gereinigt und das Produkt dieses Beispiels als gelbliches viskoses Oel erhalten und durch Elementaranalyse und SH-Gehalt definiert.

1) <u>Vorstufe:</u> 4-(β-Hydroxy-äthylmercaptomethyl)-2,6-di-t-butyl-phenol:

200 ml Aethanol, 2,6 g pulversisiertes Kaliumhydroxid, 18,9 g para-Formaldehyd und 31,2 g 2-Mercaptoäthanol werden unter

Rühren und Stickstoff bei 50°C innert 3 Stunden mit einer Lösung von 82,5 g 2,6-Di-t-butylphenol in 60 ml Aethanol versetzt und weitere 3 Stunden bei 60°C gerührt. Nach Zugabe von 2,6 ml Eisessig werden 300 ml Toluol zugefügt und die organische Phase mit 300 ml Wasser gewaschen und getrocknet. Der Abdampfrückstand wird aus Hexan umkristallisiert. Farblose Kristalle, Smp.: 60°C.

<u>Beispiel 4:</u>

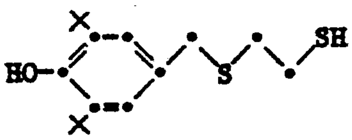

26,3 g N-(3,5-Di-t-butyl-4-hydroxybenzyl)-N,N-dimethylamin und 11,0 g Aethandithiol werden unter Stickstoff und Rühren 17 Stunden bei 100 - 110°C erhitzt. Im Dünnschichtchromatogramm sind danach nur noch Spuren des Amin-Edukts zu finden. Das Rohprodukt wird über Methylenchlorid/Wasser gereinigt, durch Vakuum-Destillation vom Lösungsmittel und Rest Aethandithiol befreit und das Produkt dieses Beispiels schliesslich säulenchromatographisch als gelbliches Oel, definiert durch SH-Titration und Elementar-Analysen, rein erhalten.

<u>Beispiel 5:</u> 3,5-Di-t-butyl-4-hydroxyphenylpropionsäure-β-mercaptoäthylamid.

14,63 g 3,5-Di-t-butyl-4-hydroxyphenylpropionsäuremethylester und 4,09 g Cysteamin (II) werden unter Stickstoff auf 230°C erhitzt und während 40 Minuten bei dieser Temperatur gehalten. Man lässt abkühlen nimmt in Aethan auf und extrahiert mit einem Ueberschuss an 1-n Salzsäure. Den Aetherextrakt wäscht man mit Natriumbicarbonatlösung (ges.) einmal. Die äthanische Lösung wird über Natriumsulfat(anhydro) getrocknet und am Rotationsverdampfer eingeengt. - Der NMR-spektroskopische ermittelte Umsatz zum Amid (V) beträgt ca. 40 %, wobei das restliche Material im wesentlichen aus Edukt (I)

besteht (DC-Kontrolle!) - Das Rohprodukt chromatographiert man an Kieselgel (Merck, 70 - 230 mesh ASTM) mit Toluol/Essigester = 1/1, wobei 0,8 g des NMR-spektroskopisch definierten Produktes isoliert werden.

1) Auf bekannte Weise erhältlich durch Erhitzen von molaren Mengen 2,6-Di-t-butylphenol und Acrylsäuremethylester ca. 6 Stunden bei 120°C in Gegenwart von 15 Mol-% Natriummethylat. Isoliert aus der Toluollösung nach Wasserwäsche. Kristalle aus Isopropanol vom Smp. 64°C.

<u>Beispiel 6:</u> 12,53 g 3-Methyl-5-t-butylphenylpropionsäuremethylester[1] (0,05 Mol) sowie 4,09 g (0,053 Mol) Cysteamin (II) werden unter Stickstoff auf 230°C erhitzt und während 75 Minuten auf dieser Temperatur gehalten. Die Aufarbeitung erfolgt wie im vorher genannten Beispiel. - Die NMR-spektroskopische Analyse des Rohprodukts zeigt, dass der Umsatz zum Amid (III) zu ca. 50 % erfolgt ist; das übrige Material bestand im wesentlichen aus Edukt (I) (AC-Kontrolle!) - Dieses Rohprodukt wird nun im Kugelrohrofen bei einer Temperatur (Ofen) 200 - 205°C/0,02 Torr. destilliert, wobei das Amid III als unflüchtiges Harz zurück bleibt und so vom Edukt (I) getrennt werden kann. Das Produkt III des Beispiels bildet ein durch NMR-spektroskopische Daten belegtes hochviskoses Oel.

1) Erhalten auf bekannte Weise analog dem Di-t-butylphenol-Edukt des vorstehenden Beispiels.

<u>Beispiel 7:</u>

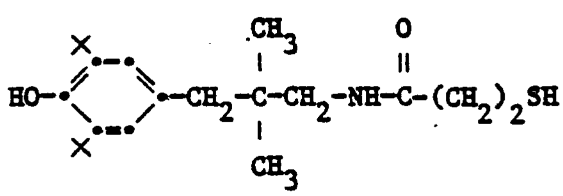

BAD ORIGINAL

58,3 g (0,2 Mol) von 3-(3,5-Di-t-butyl-4-hydroxyphenyl)-2,2-dimethyl-propylamine[1] und 17,5 ml (0,2 Mol) von 3-Mercaptopropionsäure werden bis auf 180°C geheizt. Wasser spaltet sich ab und das klare leicht gelbliche Reaktionsgemisch wird nach 4 Stunden bei 180°C abgestellt. Dieses wird bei 60°C in 100 ml n-Hexane aufgenommen, abfiltriert und die klare leicht gelbliche Lösung eingeengt.
Aspekt: leicht gelbliches Harz.
Durch El.-Analyse und -SH-Bestimmung definiert.

1) Das Amin wird auf bekannte Weise hergestellt durch katalytische Hydriertung von 2,2-Dimethyl-3-(3,5-di-t-butyl-4-hydroxyphenyl)-propionaldehyd in Gegenwart von Raney-Nickel und überschüssigem Ammoniak in Methanol. Smp.: 108°C aus Acetonitril.

Beispiel 8:

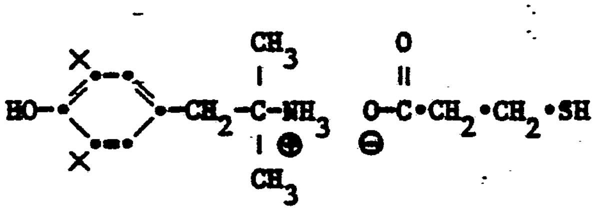

Zu 27,8 g (0,1 Mol) von 2-(3,5-Di-t-butyl-4-hydroxyphenyl)-1,1-dimethyläthylamine[1] wird bei 120°C unter $N_2$ 8,7 ml (0,1 Mol) von Mercaptopropionsäure tropfenweise zugegeben. Das Reaktionsgemisch wird nach 30 Minuten abgekühlt und das weisse Produkt mit 150 ml n-Hexane gut gewaschen. Dieses wird abgesaugt und getrocknet.
Aspekt: weisses Produkt. Fp. 210°C.

1) Hergestellt gemäss Anmerkung zum vorstehenden Beispiel.

Beispiel 9: 2-(3,5-Di-t-butyl-4-hydroxybenzyl)-malonsäure-mono-β-mercaptoäthyl-mono-methylester.

BAD ORIGINAL

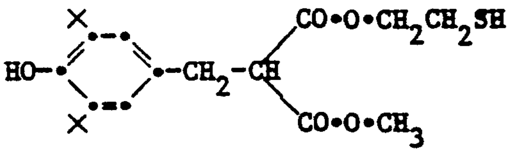

35,0 g 2-(3,5-Di-t-butyl-4-hydroxybenzyl)-malonsäuredimethylester im 350 ml Sulfierkolben vorlegen und auf 155 - 160°C erwärmen. Nach Zusatz von 0,2 ml Tetrabutylorthotitanat wird der Druck auf 250 - 300mm Hg reduziert und langsam innert 2 1/2 Stunden 18,6 g 2-Mercaptoäthanol zugetropft. Es destilliert Methanol ab. Gleichzeitig wird darauf geachtet, dass der Kp. nicht über 40 - 50 % steigt. Anschliessend wird das Reaktionsgemisch weitere 6 Stunden bei 155 - 160°C und 250 - 300 mm Hg gerührt.

Nach Abkühlen auf Raumtemperatur und belüften mit Stickstoff wird das braune Oel mit 170 ml Toluol und 3 g Tonsil aufgekocht und filtriert. Die erhaltene Lösung wird am Rotavapor eingedampft und der ölige Rückstand in 100 ml Hexan aufgenommen, mit Tonsil versetzt und klar filtriert.

Beim Abkühlen fällt das Produkt als dunkelgelbes Oel aus, welches nach Abdekantieren vom Lösungsmittel 15 Stunden bei 80°C am Hochvakuum getrocknet wird.
Durch Elementaranalyse und NMR identifiziert.

Verwendungsbeispiele

Beispiel I: 100 g Polybutadien-Latex mit 30 % Feststoffgehalt werden bei 55°C unter Stickstoff gerührt. Bei dieser Temperatur werden in der angegebenen Reihenfolge die folgenden Substanzen zugesetzt:

a) 1 g Natriumstearat in 100 ml $H_2O$,

b) 0,3 g einer Verbindung A, welche folgende Formelstruktur aufweist

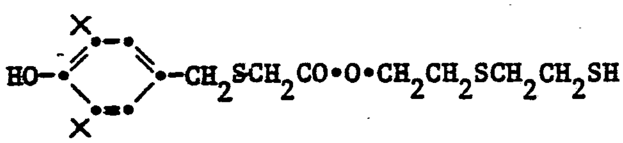 (A)

c) 0,01 g Azo-bis-isobutyronitril.

Das Gemisch wird während 10 Stunden gerührt. Anschliessend wird die Emulsion durch Zugabe von 100 ml 2,5 %iger Schwefelsäure bei 55°C koaguliert und der ausgefallene Kautschuk gründlich mit Wasser gewaschen. Nach der Filtration wird im Vakuum bei 40°C während 3 Stunden vorgetrocknet. Zur Entfernung des Wassers wird der Kautschuk dreimal durch ein kaltes Walzwerk gelassen. Dann wird bei Raumtemperatur im Vakuum während 12 Stunden getrocknet.

Der trockene Kautschuk wird in einer Heizpresse bei 60°C zu 2 mm dicken Platten verpresst (Presszeit 20 Minuten).

Die Prüfung auf Wirksamkeit des zugesetzten Additives wird durch Hitzealterung vorgenommen. Die Hitzealterung besteht im Eintauchen der Prüflinge in Silikonöl bei 160°C während 20 Minuten. Als Kriterium dient der Gelgehalt am Ende der Alterung. Dieser wird folgendermassen bestimmt:

1 g Polybutadien wird über Nacht bei Raumtemperatur in 100 ml Toluol gelöst. Die Lösung wird durch Glaswolle filtriert und die filtrierte Lösung zur Trockne eingedampft. Der Gelgehalt ergibt sich aus:

BAD ORIGINAL

$$Gel = \frac{E - W}{E} \times 100\ (\%)$$

E = Einwaage (üblich 1 g)

W = Gewicht des Eindampfrückstandes

Im vorliegenden Fall zeigen die Versuchsergebnisse der Hitzealterung, dass Gelentwicklung durch das zugesetzte Additiv A wirksam unterdrückt wird.

In einem 2. Versuch wird die günstige Wirkung des Stabilisators ebenfalls durch Alterung nachgewiesen. Die Alterung in Siliconöl erfolgt hier aber nach vorheriger 24-stündiger Extraktion der Probe in Aethanol bei Raumtemperatur.

Die Versuchsergebnisse von Beispiel I sind in den Tabellen 1 und 2 veranschaulicht. Sie zeigen, dass es sich um ein extraktionsstabiles Stabilisator-System handelt.

BAD ORIGINAL

Tabelle 1

Alterung ohne vorherige Extraktion mit Aethanol

| | Gelgehalt (%) |
|---|---|
| Ohne Stabilisator | 30 |
| Stabilisator A | 7 |

Tabelle 2

Alterung nach vorheriger Extraktion mit Aethanol

| | Gelgehalt (%) |
|---|---|
| Ohne Stabilisator | 50 |
| Stabilisator A | 8 |

Patentansprüche

1. Verbindungen der Formel I

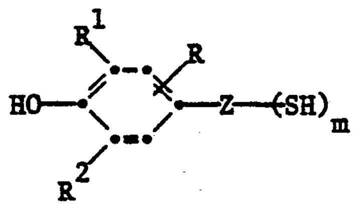

(I) ,

in der m eine der Zahlen 1 oder 2 darstellt, $R^1$ und $R^2$ gleich oder verschieden sind und einen geradkettigen oder verzweigten Alkylrest mit 1 bis 18 C-Atomen, einen Cycloalkyl-, Aryl-, Alkaryl- oder Aralkylrest bedeuten, wobei $R^2$ im Gegensatz zu $R^1$ auch für H stehen kann, in der R einen Alkylrest mit 1 bis 12 C-Atomen oder H bedeutet, in der Z $-OR^6-$, $-NH-R^6-$, $-A-(O-R^6-)_m$, $-A(-NH-R^6-)_m$, $-B-CO \cdot C_nH_{2n}-$,

$-C_qH_{2q}-$, 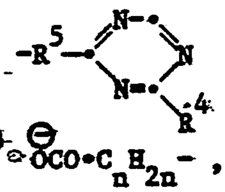 , $-C_2H_{2c}COO^{\ominus}\ {}^{\oplus}NH_3C_nH_{2n}-$, $-{}^{\oplus}NH_3\ {}^{\ominus}OCO \cdot C_nH_{2n}-$ ,

$C_2H_{2c}NH_3^{\oplus}\ {}^{\ominus}OCO \cdot C_nH_{2n}-$ ,

und nur bei m=2, 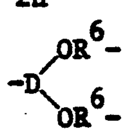 oder 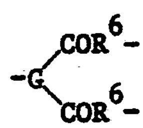 darstellt,

wobei A bei m = 1 einen der 2-wertigen Reste aus der Reihe

$-C_tH_{2t}SC_nH_{2n} \cdot CO-$, $-C_nH_{2n} \cdot COO \cdot C_tH_{2t}SC_nH_{2n}CO-$, $-C_tH_{2t}CO \cdot O \cdot C_nH_{2n}S-C_sH_{2s} \cdot CO-$,
$-OC_nH_{2n}CO-$,

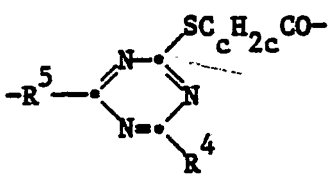

,

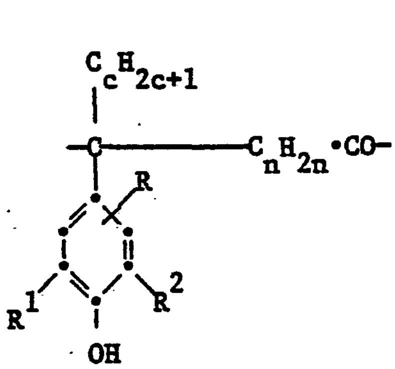 und 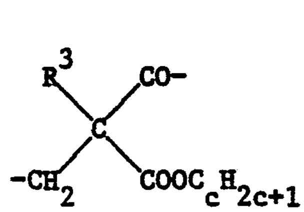

oder aber bei m = 2

einen 3-wertigen Rest

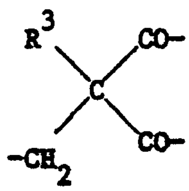

,

B einen Rest aus der Gruppe $-O-$, $-NH-$, $-C_nH_{2n}O-$, $-C_nH_{2n}NH-$,

$-C_tH_{2t}SC_nH_{2n}O-$, $-OC_cH_{2c}-$ , $-CH_2SC_nH_{2n}OC_tH_{2t}-$, $-CH_2SC_nH_{2n}-$,

$-C_cH_{2c}CONHC_nH_{2n}-$, $-C_cH_{2c}CONHC_nH_{2n}-NH-$, $-C_cH_{2c}CONH-$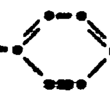$-NH-$,

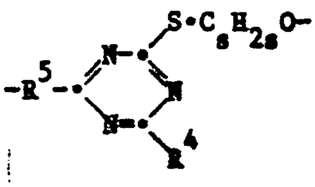

, $-C_nH_{2n}CO{\cdot}O{\cdot}C_tH_{2t}NH-$ , $-R^5-C_nH_{2n}O-$ ,

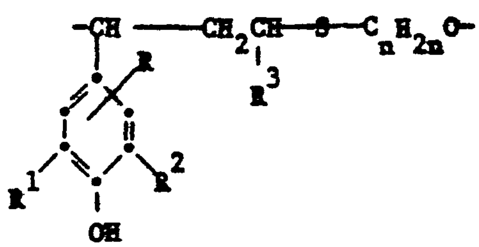

,

D den Rest

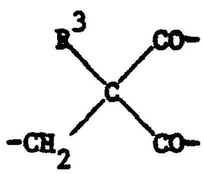

und

G den Rest

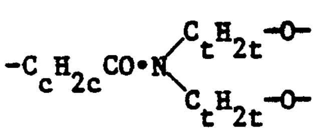

bedeuten, und wobei

c, n, r, s und t gleich oder verschieden sind und für eine Zahl von 1 bis 12 stehen, q für eine Zahl von 2 bis 12 steht, mit der Massgabe, dass die einzelnen Indizes c, n, q, r, s und t ebenfalls

gleich oder verschieden sein können, wenn dieselben mehrmals in gleichen oder verschiedenen Molekülgruppen der Verbindung vorkommen, $R^3$ einen Alkylrest mit 1 bis 18 C-Atomen, den Rest

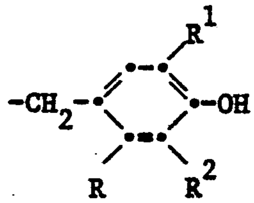 oder H und $R^4$ einen der Reste

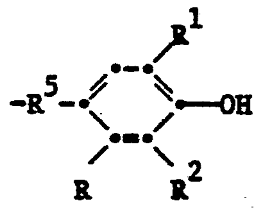 ,

$-R^5-C_p\cdot H_{2p+1}$ oder -SH bedeuten, wobei p eine Zahl von 1 bis 18 darstellt, $R^5$ für einen der Reste -NH-, -O- oder -S- und $R^6$ für einen durch Aryl- oder -OH substituierten oder unsubstituierten Alkylenrest mit 2 bis 18 C-Atomen, welcher auch durch -O- oder -S-Brücken unterbrochen sein kann, steht.

2. Verbindungen nach Anspruch 1 der Formel 1, in der m die Zahl 1 und t, n, r und s unabhängig voneinander eine der Zahlen 1, 2 oder 3 und q 3 bedeuten.

3. Verbindungen nach Anspruch 1, dadurch gekennzeichnet, dass mindestens $R^1$ oder $R^2$, vorzugsweise beide Reste, einen tert. Butylrest bedeuten.

4. Verbindungen nach Anspruch 1 der Formel I, in der m eine der Zahlen 1 oder 2 darstellt, $R^1$ und $R^2$ gleich oder verschieden sind und einen tert.Butylrest, einen Methylrest, einen Isopropylrest, einen Phenylrest oder den Rest

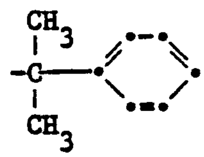

bedeuten, in der Z entweder

$-A(-OCH_2CH_2-)_m$ oder $-B-CO\cdot CH_2-$ oder $-C_qH_{2q}-$ oder $-R^5-$ 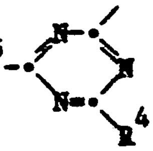

darstellt, wobei A bei m = 1 einen der 2-wertigen Reste aus der Reihe $-CH_2SCH_2CO-$, $-(CH_2)_3SCH_2CO-$, $-CH_2CO\cdot O(CH_2)_3-SCH_2CH_2CO-$ und $-OCH_2CO-$ oder aber bei m = 2 einen 3-wertigen Rest

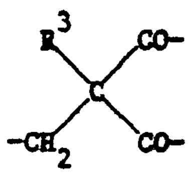

und B einen Rest aus der Gruppe

$-R^5-$ 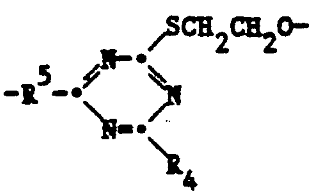 oder $-R^5-CH_2CH_2O-$

bedeuten und wobei q für eine der Zahlen 2 oder 3, $R^3$ für einen Alkylrest mit 1 bis 18 C-Atomen, $R^4$ für einen der Reste

$-R^5-$ 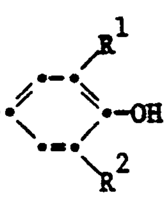 oder $-R^5-C_pH_{2p}$ ,

wobei p eine Zahl von 1 bis 6 bedeutet, und $R^5$ für einen der Reste -NH-, -O- oder -S- steht.

5. Verbindungen nach Anspruch 1 der Formel I, in der Z einen der Reste

$-C_qH_{2q}-$ ,

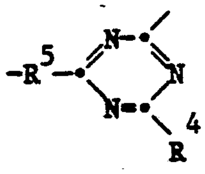

,

$-OR^6-$ ,

$-C_cH_{2c}COO^{\ominus} \cdots {}^{\oplus}NH_3\ C_nH_{2n}-$

oder

$-{}^{\oplus}NH_3\ {}^{\ominus}OCOC_nH_{2n}-$

darstellt.

6. Verbindungen nach Anspruch 1 der Formel I, in der Z den Rest $-A-OR^6-$ bedeutet, wobei A für einen der Reste

$-C_tH_{2t}COOC_nH_{2n}S-C_sH_{2s}\cdot CO-$ ,

$-C_tH_{2t}SC_nH_{2n}CO-$ ,

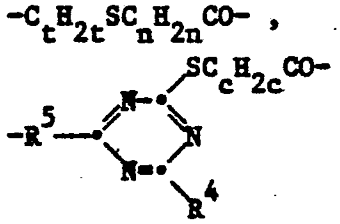

$-OC_nH_{2n}CO-$ oder

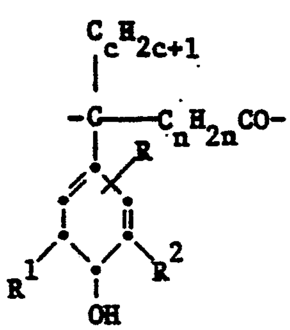

steht.

7. Verbindungen nach Anspruch 1 der Formel I, in der Z den

Rest 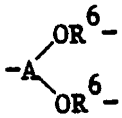 bedeutet, wobei A für den 3-wertigen Rest

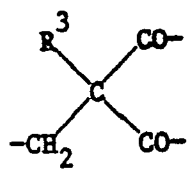 steht.

8. Verbindungen nach Anspruch 1 der Formel I, in der Z den Rest

$-B-CO \cdot C_nH_{2n}-$ bedeutet, wobei

B für einen der Reste -O-, -NH-,

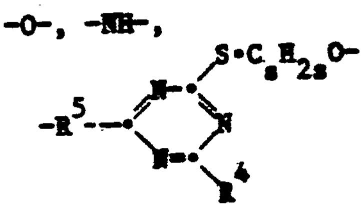 ,

$-C_tH_{2t}SC_nH_{2n}O-$ ,

$-C_cH_{2c} \cdot CO \cdot NHC_nH_{2n}-NH-$ ,

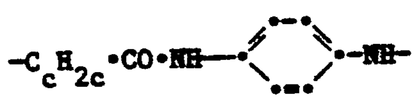 ,

$-C_nH_{2n}NH-$ ,

$-C_nH_{2n}CO \cdot O \cdot C_tH_{2t}NH-$ und

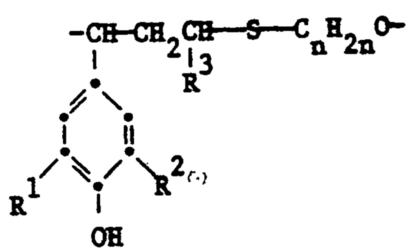

steht.

9. Verbindungen nach Anspruch 1 der Formel I, welche folgende Struktur aufweist:

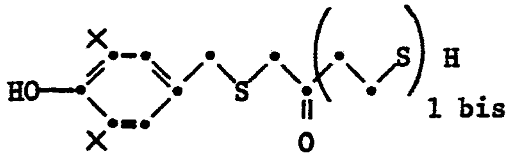

1 bis 4 mal

10. Verbindung nach Anspruch 1 der Formel I, welche folgende Struktur aufweist:

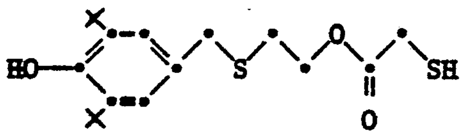

11. Verbindung nach Anspruch 1 der Formel I, welche folgende Struktur aufweist:

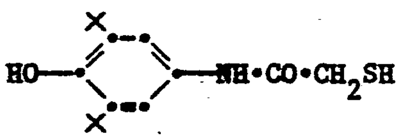

12. Verwendung von Verbindungen nach Anspruch der Formel I oder bekannter Verbindungen der Formeln II oder III

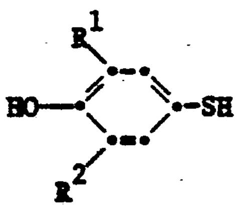 (II) 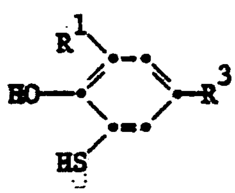 (III) ,

als Stabilisatoren für organisches Material.

13. Verwendung nach Anspruch 12, dadurch gekennzeichnet, dass man die bekannten Stabilisatoren der Formel II oder III als Stabilisatoren einsetzt.

14. Verwendung nach Anspruch 12, dadurch gekennzeichnet, dass man die verbindungen der Formel I, II und III in Gegenwart eines Radikalbildners auf ein Polymeres aufpfropft.

15. Durch Verbindungen der Formeln I, II und III nach Anspruch 12 stabilisierte organische Polymere.

16. Stabilisierte Polymere nach Anspruch 15, dadurch gekennzeichnet, dass sie als Stabilisatoren Verbindungen der Formel I nach Anspruch 1 enthalten.

17. Stabilisierte Polymere nach Anspruch 15, dadurch gekennzeichnet, dass sie die Stabilisatoren aufgepfropft enthalten.

FO 7.3/HP/rz*/rö*

0035472

Nummer der Anmeldung

Europäisches Patentamt

# EUROPÄISCHER RECHERCHENBERICHT

EP 81 81 0059

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| D,X | DE - A - 2 659 406 (THE GOODYEAR TIRE & RUBBER CO.)<br>* Seite 10; Beispielen 1,2,3,4 *<br>--<br>US - A - 4 021 468 (CIBA GEIGY CO.)<br>* Spalte 10, Tabelle 1 *<br>--------- | 1<br><br><br>1 | C 07 C 149/20<br>149/18<br>C 08 K 5/37 |
| | | | RECHERCHIERTE SACHGEBIETE (Int. Cl.3) |
| | | | C 07 C 149/20<br>149/8 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Den Haag | 1-06-1981 | GRAMAGLIA |